# EUROPÄISCHE PATENTSCHRIFT

(11) **EP 0 635 021 B1**
(45) Veröffentlichungstag und Bekanntmachung des Hinweises auf die Patenterteilung: **09.10.1996**
(21) Anmeldenummer: 93908906.6
(22) Anmeldetag: 05.04.1993
(51) Int. Cl.: C07F 9/30, C07F 9/32

(54) **VERFAHREN ZUR HERSTELLUNG VON DERIVATEN DER BIS(AMINOMETHYL)PHOSPHINSÄURE**
PROCESS FOR PRODUCING DERIVATIVES OF BIS(AMINOMETHYL) PHOSPHINIC ACID
PROCEDE DE FABRICATION DE DERIVES D'ACIDE BIS(AMINOMETHYL)-PHOSPHINIQUE

(30) Priorität: 06.04.1992 DE 4211536
(43) Veröffentlichungstag der Anmeldung: 25.01.1995
(73) Patentinhaber: HOECHST AKTIENGESELLSCHAFT, 65926 Frankfurt am Main (DE)
(72) Erfinder: PEYMAN, Anuschirwan, D-65779 Kelkheim/Ts (DE); BUDT, Karl-Heinz, D-6233 Kelkheim (DE); SPANIG, Jörg, D-1195 Berlin (DE); LI, Jian-Qi, D-6230 Frankfurt am Main 80 (DE); STOWASSER, Bernd, D-6114 Gross-Umstadt (DE)
(86) Internationale Anmeldenummer: EP9300838
(87) Internationale Veröffentlichungsnummer: WO9320086

(56) Entgegenhaltungen:
- DE-A- 3 824 961
- FR-A- 2 380 289
- PHOSPHORUS, SULFUR, AND SILICON AND THE RELATED ELEMENTS Bd.62, Nr. 1-4, 1991 Seiten 75-81 R. TYKA ' Synthese symmetri scher und asymmetrischer Phosphins uren ' in der Anmeldung erwähnt
- TETRAHEDRON LETTERS Bd. 33, Nr. 32, 1992, OXFORD GB Seiten 4549 - 4552 A.
- PEYMAN 'C2-Symmetic Phosphinic Acid Inhibitors of HIV Protease'

## Beschreibung

α-Aminophosphonsäuren und deren Derivate erlangen, oftmals als Enzyminhibitoren, immer größere Bedeutung in der Biochemie, der pharmazeutischen Chemie und im Bereich des Pflanzenschutzes [E. Neuzil, A. Cassaigne, Exp. Ann. Biochim. Medicale 34 (1980) 165]. Zur Synthese dieser Verbindungsklasse gibt es eine ganze Palette von Verfahren, die in verschiedenen Arbeiten zusammengefaßt wurden [Kukhar und Solodenko, Russ. Chem. Rev. 56 (1987) 859, Redmoore, Topics in Phosphorus Chemistry, Vol. 11, Grayson and Griffith, Ed., Wiley 1976, 515].

Die Darstellung einer anderen Verbindungsklasse, der Bis(aminomethyl)phosphinsäure und deren Derivate, denen in der pharmazeutischen Chemie ebenfalls steigende Bedeutung zukommt (EP 0435 059 A1) ist weitaus aufwendiger und nur wenig dokumentiert. Die Synthese der Stammverbindung wurde erstmals von Meier beschrieben [L. Meier, J. Organomet. Chem. 178 (1979) 157, DE 2805074A1, s. auch Kober et al., DE 3824961A1]. Eine Möglichkeit zur Herstellung von α bzw. α,α'-substituierten Derivaten der Bis(aminomethyl)phosphinsäure wird von Tyka et al. [Phosphorus, Sulfur, and Silicon 62 (1991) 75] beschrieben. Die Methode beruht auf der Addition von unterphosphoriger Säure an Schiff'sche Basen unter Bildung von Aminoalkylphosphonigen Säuren im ersten Schritt, die dann im folgenden mit Arylidenbisamiden zu α,α'-Bis(aminoalkyl)phosphinsäuren umgesetzt werden. Dieses Verfahren ist jedoch begrenzt auf die Umsetzung mit Arylidenbisamiden und ist weiter gekennzeichnet durch mäßige Ausbeuten. Nur mäßige Ausbeuten wurden auch für die doppelte Addition von unterphosphoriger Säure an Schiff'sche Basen unter Bildung von α,α'-Bis(aminoalkyl)phosphinsäuren beobachtet.

Gegenstand der Erfindung ist ein neues Verfahren zur Herstellung von α- bzw. *α,α'*-substituierten Derivaten der Bis(aminomethyl)phosphinsäure der Formel I und deren saurer oder basischen Salze, 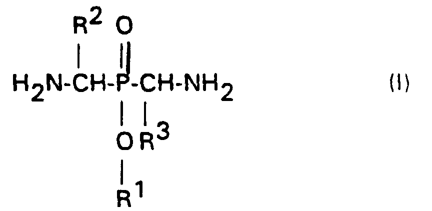 worin R¹ für R⁶ = C₁-C₁₈ Alkyl, C₂-C₁₈ Alkenyl, C₂-C₁₈ Alkinyl, C₆-C₁₂ Aryl oder C₇-C₂₀ Arylalkyl steht und Aryl oder Arylalkyl ein oder mehrfach durch Fluor, Chlor, Brom, NO₂, CN, OH, COOH, C₁-C₆ Alkyl, C₁-C₆ Alkoxy, C(O)-O-(C₁-C₆)-Alkyl, C(O)-(C₁-C₆)-Alkyl, O-C(O)-(C₁-C₆)-Alkyl substituiert sein kann, und R¹ darüber hinaus für Wasserstoff oder basische Salz reste der α- bzw. *α,α'*-substituierten Derivate der Bis(aminomethyl)phosphinsäure, insbesondere der Alkali- bzw. Erdalkalisalze, oder aber der Ammonium- bzw. Trialkylammoniumsalze, R² und R³ gleich oder verschieden sind und unabhängig voneinander für Wasserstoff, C₁-C₁₈ Alkyl, C₂-C₁₈ Alkenyl, C₂-C₁₈ Alkinyl, C₆-C₁₂ Aryl oder C₇-C₂₂ Arylalkyl steht, wobei Alkyl, Alkenyl oder Alkinyl jeweils ein oder mehrfach durch Fluor, Chlor, Brom, NO₂, NH₂, CN, OH, COOH, C(O)-O-(C₁-C₆)-Alkyl, C(O)-(C₁-C₆)-Alkyl , O-C(O)-(C₁-C₆)-Alkyl oder C₁-C₆ Alkoxy substituiert sein können, und Aryl ein oder mehrfach durch Fluor, Chlor, Brom, NO₂, CN, OH, COOH, C₁-C₆ Alkyl, C₁-C₆ Alkoxy, C(O)-O-(C₁-C₆)Alkyl, C(O)-(C₁-C₆) Alkyl , O-C(O)-(C₁-C₆) Alkyl substituiert sein kann, mit der Maßgabe, daß falls R² = Wasserstoff ist und R³ die obengenannte Bedeutung hat, R³ nicht ebenfalls für Wasserstoff stehen darf und umgekehrt.

R¹ steht bevorzugt für Wasserstoff, C₁-C₄ Alkyl, C₂-C₄ Alkenyl, Phenyl, Benzyl, Li⁺, Na⁺, K⁺, Mg²⁺, NH₄⁺, HN(C₂H₅)₂³⁺, insbesondere für Wasserstoff und C₁-C₄ Alkyl.

Besonders bevorzugt stehen R² und R³ für C₁-C₆ Alkyl, C₂-C₆ Alkenyl, C₂-C₆ Alkinyl, C₆-C₁₂ Aryl, C₇-C₁₃ Arylalkyl, wobei Aryl oder Arylalkyl ein oder mehrfach mit Chlor, Brom, -CN,O-C₁-C₃-Alkyl substituiert sein kann. Ganz besonders bevorzugt stehen R² und R³ für C₁-C₆ Alkyl, C₂-C₆ Alkenyl, C₂-C₆ Alkinyl und Benzyl.

Formel I umfaßt ferner die sauren Salze der α- bzw. α,α'-substituierten Derivate der Bis(aminomethyl)phosphinsäure, insbesondere der Hydrochloride, Hydrobromide, Hydrogensulfate, besonders bevorzugt der Hydrochloride und Hydrobromide.

### A. Synthese der Verbindungen der Formel II

Das Verfahren ist dadurch gekennzeichnet, daß zunächst die Aminogruppierungen der Bis(aminomethyl)phosphinsäure [Synthese nach L. Meier, J. Organomet. Chem. 178 (1979) 157] geschützt werden (Formel II). 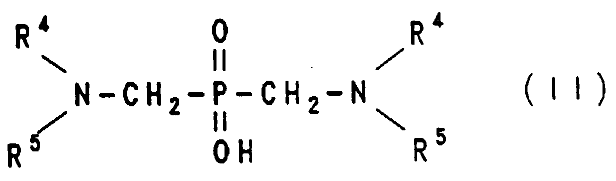 In Formel II soll NR⁴R⁵ für die geschützte Aminofunktion stehen. Als Schutzgruppen R⁴ und R⁵ können die üblichen Schutzgruppen für Aminofunktionen [Greene, Protective Groups in Organic Synthesis, Wiley 1979] in bekannter Weise eingesetzt werden, insbesondere aber die für die Peptidchemie bekannten Schutzgruppen [Bodanszky & Bodanszky, The Practice of Peptide Synthesis, Springer 1984] in der für Aminosäuren allgemein bekannten Weise eingeführt werden. Besonders bevorzugt ist die Umsetzung von Bis(aminomethyl)phosphinsäure mit 2 bis 2.5 Equivalenten Di-tert-butylpyrocarbonat in Gegenwart eines geringen Überschusses an Base (bezogen auf die Aminogruppen), vorzugsweise NaOH in wässrigem Dioxan bei -30 bis + 50°C, vorzugsweise bei -10 bis + 20°C während 0.5-3 h. Die Aufarbeitung erfolgt, wie bei Bodanszky für Aminosäuren beschrieben in an sich bekannter Weise durch Einengen, Ansäuern auf pH 2-3 und Extraktion.

### B. Synthese der Verbindungen der Formel III

Die N,N'-geschützte Bis(aminomethyl)phosphinsäure II wird im nächsten Schritt mit einer Verbindung der Formel R⁶-OH in eine Verbindung der Formel III überführt. 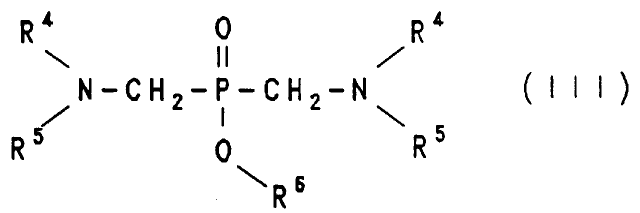 R⁴ und R⁵ sind dabei wie in Formel II definiert und R⁶ hat die in R¹ genannte Bedeutung.
R⁶ steht bevorzugt für C₁-C₄ Alkyl, C₂-C₄ Alkenyl, Phenyl, Benzyl, ganz besonders bevorzugt für C₁-C₄ Alkyl.

Die Veresterung erfolgt durch Umsetzung der geschützten Phosphinsäure II, den Verbindungen R⁶-OH (wobei R⁶ wie oben definiert ist) und einem geeigneten Kupplungsreagens. Die Bedingungen für die Veresterung richten sich nach den allgemein bekannten Vorschriften zur Veresterung von Phosphonsäuren, wie sie z. B. in Houben-Weyl (Band 12/1 & E2) aufgeführt sind. Alternativ können Kupplungsreagentien, wie sie zur Veresterung N-geschützter α-Aminocarbonsäuren eingesetzt werden [Janin et al., Tetrahedron Lett. 28 (1987) 1661], verwendet werden oder es können auch Kupplungsreagentien, die zur Synthese von Phosphorsäuretriestern in der DNA-Synthese Anwendung finden [Sonveaux, Bioorg. Chem. 14 (1986) 274], eingesetzt werden.

Besonders bevorzugt ist die Umsetzung von II mit 1-10 Equivalenten R⁶-OH und 1-2, bevorzugt 1-1,2 Equivalenten Dicyclohexylcarbodiimid (DCC) in einem geeigneten organischen Lösungsmittel, bevorzugt THF, bei Temperaturen von 0-100°C, bevorzugt bei 40-70°C (in THF bei 40-67°C) während 0,5 bis 48 h. Die anfallenden Ester III werden in an sich bekannter Weise durch Einengen, Abfiltrieren von N,N'-Dicyclohexylharnstoff, Kristallisation, Chromatographie an Kieselgel gereinigt.

Ebenfalls besonders bevorzugt ist die Umsetzung von II mit R⁶-OH in Gegenwart von Alkylchloroformaten oder Alkenylchloroformaten, die zur Veresterung von α-Aminocarbonsäuren eingesetzt werden [Janin et al., Tetrahedron Lett. 28 (1987) 1661]. Diese Methode läßt sich überraschenderweise auch auf die Veresterung der Phosphinsäuren II übertragen. Dazu wird II umgesetzt mit 1-2 Equivalenten, bevorzugt 1 bis 1,3 Equivalenten Alkyl- bzw. Alkenylchloroformat, bevorzugt Isobutyl- oder Isopropenylchloroformat in Gegenwart von 1 bis 2, bevorzugt 1 bis 1,2 Equivalenten Trialkylamin, bevorzugt Triethylamin (TEA) oder Diisopropylethylamin (DIPEA) und einer katakytischen Menge Dimethylaminopyridin (DMAP), bevorzugt 0,1 Equivalenten DMAP, sowie 1-10 Equivalenten R⁶-OH in einem geeigneten organischen Lösungsmittel, bevorzugt Methylenchlorid (CH₂Cl₂), Chloroform (CHCl₃), Tetrahydrofuran (THF), Acetonitril, Dimethylformamid (DMF), Dimethylsulfoxid (DMSO), Dioxan, Toluol, Benzol, Essigsäureethylester (EE), besonders bevorzugt CH₂Cl₂ oder CHCl₃ und Toluol bei Temperaturen von -10 bis +50°C, bevorzugt 0-10°C während 1-12 h. Die Aufarbeitung und Reinigung erfolgt in an sich bekannter Weise wie bei der Veresterung von α-Aminosäuren beschrieben [Janin et al., Tetrahedron Lett. 28 (1987) 1661] durch Extraktion, Kristallisation, Chromatographie an Kieselgel.

Die Synthese der Verbindungen III, insbesondere der Verbindungen III in denen R⁴ für Benzyl und R⁵ für Wasserstoff, steht kann auch nach einem alternativen Verfahren erfolgen. Die zugehörige Stammverbindung Bis(benzylaminomethyl)-phosphinsäure ist schon in der Literatur beschrieben worden, nicht jedoch deren Ester [L. Meier, J. Organomet. Chem. 178 (1979) 157, DE 2805074A1]. Zur Herstellung dieser Verbindungen geht man von Bis(chlormethyl)phosphinsäurechlorid aus, welches ebenfalls von Meier beschrieben wurde [L. Meier, J. Organomet. Chem. 178 (1979) 157, DE 2805074A1]. Dieses wird durch Umsetzung mit mindestens einem Equivalent R⁶-OH (R⁶-OH wie oben definiert) in einem geeigneten organischen Lösungsmittel, bevorzugt Toluol, Benzol, CH₂Cl₂, CHCl₃, THF, Ether bei Temperaturen von 0 bis 100°C, bevorzugt bei 10 bis 40°C zu dem Bis(chlormethyl)phosphinsäureester der Formel IX

(ClCH₂)₂P(O)OR⁶ (IX)

umgesetzt. Die Reaktion kann auch in Gegenwart eines Equivalents Base, bevorzugt Trialkylamin, besonders bevorzugt Triethylamin (TEA) oder Diisopropylethylamin (DIPEA) und auch in Gegenwart einer katalytischen Menge Dimethylaminopyridin (DMAP), bevorzugt 0,1 Equivalenten DMAP, erfolgen. Wenn R⁶-OH für einen niedersiedenden Alkohol, z. B. Ethanol, steht, so kann dieser selbst als Lösungsmittel dienen. Die gebildeten Ester werden in an sich bekannter Weise durch Filtration, Einengen, Kristallisation, Destillation bzw. Chromatographie an Kieselgel gereinigt.
Zur Herstellung der Bis(benzylaminomethyl)phosphinsäureester wird mit einem Überschuß zur stöchiometrisch erforderlichen Menge Benzylamin bei Temperaturen von 0 bis 110 °C umgesetzt. Die Produkte werden in an sich bekannter Weise durch Filtration, Einengen, Kristallisation, Destillation bzw. Chromatographie an Kieselgel gereinigt. Vorzugsweise werden die
Bis(benzylaminomethyl)phosphinsäureester-Hydrochloride isoliert.

### C. Synthese der Verbindungen der Formel IV

Im folgenden Schritt werden die Aminofunktionen des Esters der Formel III entschützt, wobei die Bis(aminomethyl)phosphinsäureester der FormellV bzw. deren saure Salze entstehen. 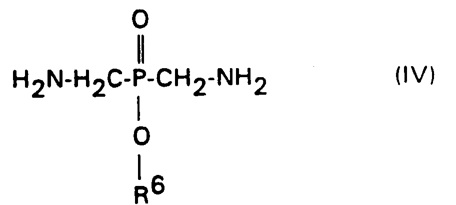 Die Entschützung erfolgt nach an sich bekannten Verfahren [Greene, Protective Groups in Organic Synthesis, Wiley 1979, Bodanszky & Bodanszky, The Practice of Peptide Synthesis, Springer 1984], je nach eingesetzter Schutzgruppe. So wird z. B. die tert.-Butyloxycarbonyl-Schutzgruppe durch Behandlung mit einer gesättigten Lösung von HCI in Dioxan oder Methanol bei 20-40°C während 10-30 Minuten und anschließendem Einengen entfernt.
Benzylschutzgruppen werden durch katalytische Hydrierung (5% Pd/C oder PtO₂ oder Pt/C) bei Normaldruck und bei 10 - 70°C , vorzugsweise 20 - 40°C, entfernt. Besonders geeignete Lösemittel sind dabei Ethanol, Ethanol/Eisessig oder Eisessig.

### D. Synthese der Verbindungen der Formel V

Der entstandene Bis(aminomethyl)phosphinsäureester IV wird im folgenden Schritt zum Bis(iminomethyl)phosphinsäureester V umgesetzt. 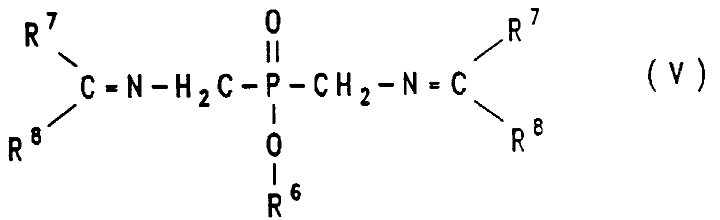 Dabei sind R⁷ und R⁸ gleich oder verschieden und stehen für Wasserstoff, C₁-C₂₀ Alkyl, C₂-C₂₀ Alkenyl, C₂-C₂₀ Alkinyl, C₆-C₁₂-Aryl. R⁷ und R⁸ können auch gemeinsam ein Ringsystem bilden, beispielsweise ein C₅-C₃₀-Alkyl-Ringsystem, ein C₅-C₃₀-Alkenyl-Ringsystem oder ein C₉-C₃₀-Alkylaryl-Ringsystem, wobei die Ringsysteme mono-, bi- oder tricyclisch sein können. In diesem Fall stehen R⁷ und R⁸ beispielsweise für die Gerüste von Cyclopentanon, Cyclohexanon, Fluorenon, Anthron, Campher, Menthon, Pulegon, Carvon, Caron, Verbenon.

Bevorzugt sind Verbindungen V, in denen R⁷ und R⁸ für Phenyl/Phenyl, Phenyl/Methyl, Phenyl/Wasserstoff oder für das Camphergerüst stehen. Besonders bevorzugt sind Verbindungen V, in denen R⁷ und R⁸ für Phenyl/Phenyl stehen.
a. Die Synthese der Bis(iminomethyl)phosphinsäureester V erfolgt durch Umsetzung von IV (bevorzugt der Hydrochlorid bzw. Hydrobromidform) mit 2 bis 3 Equivalenten (bevorzugt mit 2 bis 2,2 Equivalenten) der Verbindung VI (R⁷ und R⁸ wie oben definiert) in einem 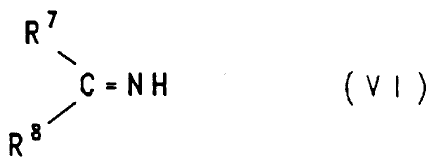 geeigneten wasserfreien organischen Lösungsmittel (bevorzugt CH₂Cl₂, CHCl₃, THF, Acetonitril, Dimethylformamid , Dimethylsulfoxid , Dioxan, Toluol, Benzol, Essigsäureethylester, besonders bevorzugt CH₂Cl₂ und CHCl₃, Toluol und Benzol, ganz besonders bevorzugt CH₂Cl₂ und CHCl₃) bei Temperaturen von 0 bis 100°C (bevorzugt 10-30°C) während 1 bis 48 h (bevorzugt 3 bis 24 h). Die Reaktion wird vorzugsweise unter Inertgas, z.B. Stickstoff oder Argon, durchgeführt. Die Aufarbeitung und Reinigung der Verbindungen V erfolgen nach allgemein üblichen Verfahren d.h. Filtration, Extraktion, Trocknen, Umkristallisieren, Chromatographie. Beispielsweise wird nach Filtration und Einengen der Lösung abermals in einem unpolaren Lösungsmittel, bevorzugt Ether, aufgenommen, wieder filtriert, getrocknet, eingeengt und an Kieselgel chromatographiert, bzw. umkristallisiert.
b. Alternativ lassen sich die Bis(iminomethyl)phosphinsäureester V herstellen durch Umsetzung der Bis(aminomethyl)phosphinsäureester IV mit 2 bis 3 Equivalenten (bevorzugt 2 bis 2,2 Equivalenten) der Verbindung VII (R⁷ und R⁸ wie oben definiert) und einer katalytischen Menge Säure (bevorzugt p-Toluolsulfonsäure) in einem geeigneten 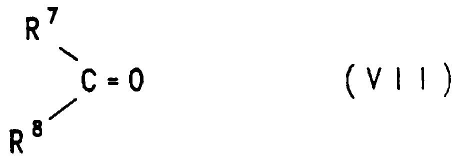 wasserfreien organischen Lösemittel (bevorzugt CH₂Cl₂, CHCl₃, THF, Acetonitril, Dimethylformamid, Dimethylsulfoxid , Dioxan, Toluol, Benzol, besonders bevorzugt CH₂Cl₂ und CHCl₃, Toluol und Benzol, ganz besonders bevorzugt Toluol und Benzol) bei Temperaturen von 0 bis 120°C (bevorzugt unter Sieden) während 1 bis 48 h (bevorzugt 10 bis 24 h) unter wasserabscheidenden Bedingungen. Bedingungen, die zur Entfernung von Wasser aus dem Reaktionsgemisch dienen, sind allgemein bekannt, bevorzugt sind Wasserabscheider und Soxleth-Apparatur mit Molekularsieb. Die Aufarbeitung und Reinigung der Verbindungen V erfolgen nach allgemein üblichen Verfahren Filtration, Extraktion Trocknen, Umkristallisieren, Chromatographie. Vorzugsweise wird zur Reinigung an Kieselgel chromatographiert.

### E. Synthese der Verbindungen der Formel VIII

Im nächsten Schritt werden die Bis(iminomethyl)phosphinsäureester V alkyliert, woraus die α,α'-substituierten Bis(iminoalkyl)phosphinsäureester VIII resultieren. 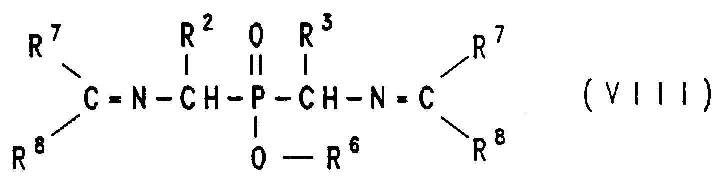 Dabei sind R², R³ und R⁶ wie in Formel I, R⁷ und R⁸ wie in Formel V definiert.

Durch den Einsatz chiraler Reste R⁷ bzw. R⁸ bzw. R⁷ und R⁸ läßt sich die Alkylierung stereoselektiv durchführen. Beispielhaft stehen in diesem Fall R⁷ und R⁸ für [+] oder [-] Campher, [+] oder [-] Menthon, [+] oder [-] Pulegon, [+] oder [-] Carvon, [+] oder [-] Verbenon.
e₁) Zur Herstellung von Verbindungen der Formel VIII, worin R² gleich R³, jedoch ungleich Wasserstoff ist, werden die Bis(iminomethyl)phosphinsäureester V in einem geeigneten absoluten organischen Lösemittel (bevorzugt THF, Ether, n-Pentan, n-Hexan, n-Heptan, besonders bevorzugt THF) gelöst und, vorzugsweise unter Inertbedingungen (besonders bevorzugt unter Argon) bei Temperauren von -90 bis +30°C (vorzugsweise -80 bis -50°C) mit 2 bis 2,2 Equivalenten einer geeigneten Base, bevorzugt Butyllithium, Lithiumdiisopropylamin (LDA), Natriumhydrid, Natriumamid, Kalium-tert.-butylat, Komplexe Basen (Natriumamid-R¹³ONa, wobei R¹³ für C₂-C₆-Alkyl oder CH₃CH₂OCH₂CH₂ steht) während 5-30 Minuten umgesetzt. Danach werden, ebenfalls bei vorzugsweise -80 bis -50°C, 2 bis 5 (bevorzugt 2 bis 2,2) Equivalente der Verbindung R³-X zugegeben. Dabei ist R³ wie in Formel I definiert, jedoch ungleich Wasserstoff, X steht für eine Abgangsgruppe, bevorzugt für Chlor, Brom, Jod, O-p-Toluolsulfonat, O-Trifluormethylsulfonat, O-Methylsulfonat, besonders bevorzugt für Chlor, Brom, Jod. Die Reaktion wird noch 2 bis 48 h bei -80 bis +30°C (vorzugsweise -80 bis -40°C) gerührt. Zur Aufarbeitung und Reinigung werden allgemein bekannte Methoden verwendet, wie Filtration, Extraktion, Umkristallisieren und Chromatographie. Beispielsweise wird das Reaktionsgemisch eingeengt, zwischen Wasser und Ether verteilt, die organische Phase in allgemein bekannter Weise getrocknet, die diastereomeren Produkte an Kieselgel chromatographiert.
e₂) Zur Herstellung von Verbindungen VIII, in denen R² für Wasserstoff steht und R³ wie in Formel I definiert ist, wird wie in e₁) vorgegangen, jedoch werden lediglich 1 bis 1,2 Equivalente Base und 1 bis 1,2 Equivalente R³-X eingesetzt. Die Reaktionsdauer beträgt 5 Minuten bis 3 Stunden.
e₃) Zur Herstellung von Verbindungen VIII, in denen R² und R³ wie in Formel I definiert sind, jedoch beide ungleich Wasserstoff sind, werden die nach e₂) erhaltenen Verbindungen der Formel VIII unter den in e₁) genannten Bedingungen mit R²-X anstelle von R³-X umgesetzt. Allerdings werden lediglich 1 bis 1,2 Equivalente Base und 1 bis 1,2 Equivalente R²-X eingesetzt. Die Reaktionsdauer beträgt 10 Minuten bis 48 Stunden.

Die in e₁) bis e₃) beschriebene Alkylierung der Bis(iminomethyl)phosphinsäureester ist besonders bevorzugt.

f. Alternativ lassen sich die Verbindungen VIII auch durch Phasen-Transfer-Katalyse erhalten.
f₁) Zur Herstellung von Verbindungen, worin R² gleich R³, jedoch ungleich Wasserstoff ist, wird eine Mischung aus V , einer katalytischen Menge R⁹R¹⁰R¹¹R¹²NZ, 2 bis 3 Equivalenten (bevorzugt 2 bis 2,5 Equivalenten) R³-X und 2 bis 25 Equivalente, (bevorzugt 8 bis 20) Equivalente eines Alkali- oder Erdalkalihydroxids, insbesondere KOH oder NaOH, und einem nicht mit Wasser mischbaren Lösemittel, bevorzugt CH₂Cl₂, Toluol, Benzol, 1,1,2,2-Tetrachlorethan, besonders bevorzugt CH₂Cl₂ und Toluol, bei Temperaturen von 0 bis 50°C, bevorzugt 10 bis 30°C, 1-24 h gerührt.
   Dabei steht N neben Stickstoff auch für Phosphor, bevorzugt jedoch für Stickstoff; R⁹, R¹⁰, R¹¹ und R¹² sind gleich oder verschieden und bedeuten unabhängig voneinander C₁-C₂₀-Alkyl, C₂-C₂₀-Alkenyl, C₂-C₂₀-Alkinylk C₆-C₁₂-Aryl oder C₇-C₂₀-Arylalkyl, bevorzugt stehen sie für C₃-C₁₆-Alkyl, besonders bevorzugt für n-Butyl; Z steht für ein Anion eines anorganischen Salzes wie Chlorid, Bromid, Jodid, Hydrogensulfat, besonders bevorzugt für Chlorid, Bromid und Hydrogensulfat; bevorzugt werden 0,03 bis 0,3, besonders bevorzugt 0,05 bis 0,15 Equivalente des Katalysators R⁹R¹⁰R¹¹R¹²NZ eingesetzt. R³-X ist dabei wie oben definiert.
   Zur Aufarbeitung und Reinigung werden allgemein bekannte Methoden verwendet, wie Filtration, Extraktion, Umkristallisieren und Chromatographie. Üblicherweise wird filtriert, das Solvens eingeengt, der Rückstand in Ether aufgenommen, abermals filtriert um den Katalysator vollständig zu entfernen, das Produkt durch Chromatographie an Kieselgel gereinigt.
f₂) Zur Herstellung der Verbindungen VIII, in denen R² für Wasserstoff steht und R³ wie in Formel I definiert ist, wird wie in f₁) vorgegangen, jedoch werden lediglich 2 bis 10, bevorzugt 3 bis 7, Equivalente des Alkali- oder Erdalkalimetallhydroxids und 1 bis 1,2 Equivalente R³-X eingesetzt.
f₃) Zur Herstellung von Verbindungen der Formel VIII, in denen R² und R³ wie in Formel I definiert sind, jedoch beide ungleich Wasserstoff sind, werden die nach f₂ erhaltenen Verbindungen der Formel VIII unter den in f₁) genannten Bedingungen mit R²-X anstelle von R³-X umgesetzt. Allerdings werden 2 bis 10, bevorzugt 3 bis 7, Equivalente des Alkali- oder Erdalkalimetallhydroxids und 1 bis 1,2 Equivalente R²-X eingesetzt.

g) Die Alkylierungen unter Phasen-Transfer-Katalyse, die in f₁), f₂) und f₃) beschrieben sind, lassen sich auch ohne Solvens durchführen. Dazu wird wie dort beschrieben verfahren, jedoch wird ohne Lösungsmittel und mit Methyltrioctylammoniumchlorid ( z. B. Aliquat® 336) anstelle von R⁹R¹⁰R¹¹R¹²NZ gearbeitet. Zur Aufarbeitung und Reinigung werden allgemein bekannte Methoden verwendet, wie Filtration, Extraktion, Umkristallisieren und Chromatographie. Üblicherweise wird das Reaktionsgemisch in Dichlormethan aufgenommen, der Katalysator durch Zugabe von Kieselgel und Filtration entfernt, das Produkt durch Chromatographie an Kieselgel gereinigt.

### F. Synthese der Verbindungen der Formel I in denen R¹ für R⁶ steht

Zur Synthese der Verbindungen der Formel I, in denen R¹ für R⁶ steht, wird VIII in einem geeigneten organischen Lösungsmittel, bevorzugt Ether, THF, CH₂Cl₂, CHCl₃, Dioxan, mit 5-15% wässriger HCI oder HBr, bevorzugt 10% wässriger Salzsäure, während 1 bis 48 Stunden, bevorzugt 5 bis 24 Stunden, bei Temperaturen von 0 bis 50°C, bevorzugt bei 10 bis 30°C umgesetzt. Zur Aufarbeitung und Reinigung werden allgemein bekannte Methoden verwendet, wie Filtration, Extraktion, Umkristallisieren, Lyophilisierung und Chromatographie. Üblicherweise werden die Phasen getrennt, die wässrige Phase wird mit einem geeigneten Lösungsmittel, bevorzugt Ether extrahiert. Die wässrige Phase wird dann mit festem K₂CO₃ und CH₂Cl₂ für 5 bis 20 min gerührt, aus der organischen Phase wird die Verbindung der Formel I gewonnen. Zur Reinigung kann noch an Kieselgel chromatographiert werden. Die Verbindung der Formel I läßt sich durch lonenaustauschchromatographie oder durch Umsetzung mit Säuren in entsprechende Salze überführen.

### G. Synthese der Verbindungen der Formel I in denen R¹ ungleich R⁶ ist

i₁) Zur Synthese der Verbindungen der Formel I, in denen R¹ ungleich R⁶ ist, werden die Verbindung der Formel VIII oder die Verbindung der Formel I, worin R¹ für R⁶ steht, in 30% HBr oder HCI, bevorzugt HBr, in Eisessig gerührt. Die Reaktion wird bei 0-80°C, bevorzugt bei 20-70°C durchgeführt. Die Reaktionsdauer beträgt 10 bis 300 min, bevorzugt 10-45 min. Danach werden Eisessig und überschüssige HBr destillativ im Vakuum entfernt. Zur Aufarbeitung und Reinigung werden allgemein bekannte Methoden verwendet, wie Filtration, Extraktion, Umkristallisieren, Lyophilisierung und Chromatographie. X läßt sich durch lonenaustauschchromatographie oder durch Umsetzung mit Säuren in entsprechende Salze überführen.
i₂) Verbindungen der Formel I, worin R¹ ungleich R⁶ ist, lassen sich alternativ aus Verbindungen der Formel I, worin R¹ für R⁶ steht, gewinnen, die sich nach den spezifischen Eigenschaften des abzuspaltenden Restes R¹ (der für R⁶ steht) richtet, nach allgemein bekannten Verfahren. Beispielhaft seien aufgeführt die Abspaltung von R⁶ = Benzyl durch Hydrierung oder von R⁶ = Methyl oder Ethyl durch Umsetzung mit Trimethylsilylbromid bzw. Trimethylsilyljodid in einem geeigneten organischen Lösungsmittel wie etwa Dioxan bei Raumtemperatur.

### Beispiele

### Beispiel 1

### Bis-(N-tert-butoxycarbonyl-aminomethyl)-phosinsäure (1)

Zu einer Lösung von Bis-(aminomethyl)-phosphinsäure-hydrochlorid (2,00 g; 12,46 mmol) in Wasser (20 ml) und Dioxan (20 ml) werden bei 0°C unter Rühren nacheinander 1,0 M wäßrige Natronlauge (37,6 ml; 37,60 mmol) und tert-Butoxycarbonylanhydrid (5,69 g; 26,10 mmol) in Dioxan (3 ml) gegeben. Die Reaktionslösung wird auf Raumtemperatur erwärmt und nach einer Stunde unter vermindertem Druck auf ein Volumen von ca. 40 ml eingeengt. Die Lösung wird mit Ethylacetat (40 ml) versetzt und mit gesättigter wäßriger KHSO₄-Lösung auf einen pH-Wert von 2-3 angesäuert. Die abgetrennte wäßrige Phase wird mit Ethylacetat (4 x 50 ml) extrahiert. Die vereinigte organische Phase wird über Na₂SO₄ getrocknet und unter vermindertem Druck eingeengt. Man erhält die Phosphinsäure 1 (3,20 g) als Rohprodukt.

¹H-NMR (δ/ppm/)(200 MHz, CDCl₃): 1,48 (18H, s, CH₃); 3,53 (4H, bd, J 7,5 Hz, CH₂); 5,70 (1H, bs NH); 6,25 (1H, bs, NH);
MS (FAB, NBA, m/e): 325 (M+H⁺), 269 (M-55), 213 (269-56)(100%).

### Beispiel 2

### Bis-(N-tert-butoxycarbonyl-aminomethyl)-phosphinsäure-ethylester (2)

Zu einer unter Rückfluß siedenden Lösung der Phosphinsäurre 1 (270 mg, 0,83 mmol) in trockenem THF (4 ml) und abs. Ethanol (0,30 ml, 5,15 mmol) wird DCC (190 mg, 0,92 mmol) in THF (4 ml) innerhalb von 4 Minuten zugetropft. Nach 1,5 Stunden (Kontrolle per DC) wird die Reaktionslösung auf Raumtemperatur abgekühlt und filtriert. Der Filterrückstand wird mit Ether gewaschen. Das Filtrat wird mit Wasser (2 x 20 ml) extrahiert. Die organische Phase wird über Na₂SO₄ getrocknet und unter vermindertem Druck eingeengt. Nach säulenchromatographischer Reinigung an Kieselgel (Ethylacetat/Heptan = 3/2) erhält man den Phosphinsäureester 2 (100 mg, 34% d.Th.). Fₚ 117,5-120°C.

¹H-NMR (δ/ppm/)(200 MHz, CDCl₃): 1,30 (3H, t, J 7 Hz, CH₂CH₃); 1,45 (18H, s, (CH₃)₃C); 3,23 (2H, dt, J 15 und 5 Hz, CH₂); 3,84 (2H, m, CH₂); 4,18 (2H, dq, J 6 und 6 Hz, OCH₂); 5,48 (2H, bs, NH);
MS (FAB, NBA, m/e): 353 (M+H⁺); 297 (M-55); 241 (297-56)(100%).

### Beispiel 3

### Bis-(N-tert-butoxycarbonyl-aminomethyl)-phosphinsäure-ethylester (2)

Zu einer Lösung der Phosphinsäure 1 (3,200 g, 9,88 mmol) in einem Gemisch von Methylenchlorid (50 ml), Ethanol (3,00 ml, 51,59 mmol), Triethylamin (1,40 ml, 10,06 mmol), und N,N-Dimethylpyridin (10 mg) wird bei 0°C unter Rühren Chlorameisensäure-iso-butyl-ester (1,50 ml, 11,48 mmol) zugetropft. Nach 90 Minuten wird die Reaktionslösung mit gesättigter wäßrigger Ammoniumchlorid-Lösung (30 ml) und gesättigter wäßriger KHCO₃-Lösung (30 ml) extrahiert. Die organische Phase wird über Na₂SO₄ getrocknet und unter verminderten Druck eingeengt. Nach säulenchromatographischer Reinigung an Kieselgel (Ethylacetat/Heptan = 3/2) erhält man den Phosphinsäureester 2 (700 mg, 20% d.Th.). Spektroskop. Daten s. o..

### Beispiel 4

### Bis-(N-Diphenylmethylen-aminomethyl)-phosphinsäure-ethylester (3)

Die tert-Butoxycarbonyl-Schutzgruppe des Phosphinsäure-ethyl-esters 2 (100 mg, 0,28 mmol) wird mit 3N methanolischer Salzsäure (30 ml) abgespalten (Kontrolle per DC). Die Reaktionslösung wird unter vermindertem Druck eingeengt. Zu der Suspension des Rohprodukts in Methylenchlorid (20 ml) wird unter einer Stickstoffatmosphäre Diphenylmethanimin (0,100 ml, 0,60 mmol) zugetropft. Die Reaktionslösung wird nach 16 Stunden Rühren bei Raumtemperatur filtriert. Das Filtrat wird unter verminderetem Druck eingeengt. Das Rohprodukt wird in Ether aufgenommen, filtriert und mit Wasser (2 x 20 ml) extrahiert. Die organische Phase wird über Na₂SO₄ getrocknet und unter vermindertem Druck eingeengt. Nach säulenchromatographischer Reinigung an Kieselgel (Ethylacetat/ Heptan = 1/1) erhält man den Diphenylmethylen-phosphinsäureester 3 (71 mg, 53% d.Th).

¹H-NMR (δ/ppm/)(200 MHz, CDCl₃): 1,30 (3H, t, J 7 Hz, CH₃); 4,10 (2H, d, J 14 Hz, CH₂N); 4,14 (4H, m, OCH₂); 7,17-7,63 (20H, m, H_{arom.});
MS (FAB, NBA, m/e): 481 (M+H⁺); 389; 327; 253; 209 (100%).

### Beispiel 5

### (N-Diphenylmethylen-aminomethyl)-(N-diphenylmethylen-1-amino-2-phenyl-ethyl)-phosphinsäure-ethylester (4)

Zu einer bei -78°C unter einer Argonatmosphäre gerührten Lösung von Phosphinsäureethylester 3 (200 mg; 0,42 mmol) in THF (10 ml) wird eine 1,53 N Butyllithium-Lösung (0,3 ml; 0,46mol) in Hexan zugtropft. Nach 5 Minuten wird zu der tiefroten Reaktionslösung Benzylbromid (0,06 ml; 0,51 mmol) zugetropft. Nach 2 h wird die Reaktionslösung auf Raumtemperatur erwärmt und nach 15 Stunden mit Ether verdünnt. Die Lösung wird mit gesättigter wäßriger Ammoniumchlorid-Lösung extrahiert (2 x 20 ml). Die organische Phase wird über Na₂SO₄ getrocknet und unter vermindertem Druck eingeengt. Nach säulenchromatographischer Reinigung an Kieselgel (Ethylacetat/Heptan = 2/3) erhält man den monoalkylierten Phosphinsäurester 4 (187 mg; 79% d.Th.).

¹H-NMR (δ/ppm/)(200 MHz, CDCl₃): 1,30 (3H, t, J 7 Hz, CH₃); 3,30 (dd, J 6 und 6 Hz, CH₂Ph); 3,90-4,15 (5H, m, CHN, CH₂N, OCH₂); 6,90-7,60 (25H, m, H_{arom.});
MS (FAB, NBA, m/e): 571 (M+H⁺), 284 (100%).

### Beispiel 6

### Bis-(N-Diphenylmethylen-1-amino-2-phenyl-ethyl)-phosphinsäure-ethylester (5)

Zu einer bei -78°C unter einer Argonatmosphäre gerührten Lösung von Phosphinsäureethylester 3 (1000 mg; 2,08 mmol) in THF (35 ml) wird eine 1,53 N Butyllithium-Lösung (3,0 ml; 4,59 mmol) in Hexan zugtropft. Nach 10 Minuten wird zu der tiefroten Reaktionslösung Benzylbromid (1,00 ml; 8,42 mmol) zugetropft. Nach 70 Stunden Rühren bei -70°C wird die Reaktionslösung mit Ether verdünnt und mit gesättigter wäßriger Ammoniumchlorid-Lösung extrahiert. Die organische Phase wird über Na₂SO₄ getrocknet und unter vermindertem Druck eingeengt. Nach säulenchromatographischer Reinigung an Kieselgel (Ethylacetat/Heptan = 2/3) erhält man die bisalkylierten Phosphinsäureester 5 (1303 mg, 95% d.Th.).

¹H-NMR (δ/ppm/)(200 MHz, CDCl₃): 1,35 (3H, t, J 7 Hz); 3.00 (2H, m, CH₂Ph); 3,49 (2H, m, CH₂Ph); 4,15-4,30 (4H, m, OCH₂ + 2 x CH-CH₂Ph); 6,90-7,60 (30H, m, H_{arom.});
MS (FAB, NBA, m/e): 661 (M+H⁺); 284 (100%).

### Beispiel 7

### Bis-(N-Diphenylmethylen-1-amino-ethyl)-phosphinsäure-ethylester (6)

In Analogie zum Beispiel 6 wurden aus dem geschützten Phosphinsäureester 3 und Methyljodid die bisalkylierten Phosphinsäureester 6 mit einer Ausbeute von 83% hergestellt.

MS (FAB, NBA, m/e): 509 (M+H⁺).

### Beispiel 8

### Bis-(N-Diphenylmethylen-1-amino-but-3-inyl)-phosphinsäure-ethylester (7)

In Analogie zum Beispiel 6 wurden aus dem geschützten Phosphinsäureester 3 und Propargylbromid die bisalkylierten Phosphinsäureester 7 mit einer Ausbeute von 81% hergestellt.

MS (FAB, NBA, m/e): 557 (M+H⁺).

### Beispiel 9

### Bis-(N-Diphenylmethylen-1-amino-4-phenyl-but-3-enyl)-phosphinsäure-ethylester (8)

In Analogie zum Beispiel 6 wurden aus dem geschützten Phosphinsäureester 3 und Cinnamylbromid die bisalkylierten Phosphinsäureester 8 mit einer Ausbeute von 76% hergestellt.

MS (FAB, NBA, m/e): 713 (M+H⁺).

### Beispiel 10

### Bis-(N-Diphenylmethylen-1-amino-3-brom-but-3-enyl)-phosphinsäure-ethylester (9)

In Analogie zum Beispiel 6 wurden aus dem geschützten Phosphinsäureester 3 und 2,3-Dibrompropen die bisalkylierten Phosphinsäureester 9 mit einer Ausbeute von 71 % hergestellt.

MS (FAB, NBA, m/e): 717, 719, 721 (M+H⁺).

### Beispiel 11

### Bis-(1-Amino-2-phenyl-ethyl)-phosphinsäure-ethylester-hydrochlorid (10)

Zu einer Lösung des bisalkylierten Phosphinsäureesters 5 (1161 mg, 1,76 mmol) in Ether (30 ml) wird unter Rühren bei Raumtemperatur eine ca. 10-proz. wäßrige Salzsäure (30 ml) gegeben. Nach 24 Stunden wird die organische Phase abgetrennt und die wäßrige Phase mit Ethylacetat (20 ml) extrahiert. Nach Gefriertrocknung erhält man das Phosphinsäureester-hydrochlorid 10 (690 mg, 97% d.Th.).

¹H-NMR (δ/ppm/)(200 MHz, DMSO-d₆): 1,15 (3H, m,
CH₃/Diastereomerengemisch/); 3,05 (4H, m, CH₂Ph); 3,93 (1H, m, CH); 4,14-4,45 (3H, m, CH + OCH₂); 7,20-7,45 (10H, m, H_{arom.}); 8,70 (6H, bs, NH₃+);; MS (FAB, NBA, m/e): 333 (M+H+); 214; 120 (100%).

### Beispiel 12

### Bis-(1-Amino-ethyl)-phosphinsäure-ethylester-hydrochlorid (11)

In Analogie zum Beispiel 11 erhält man aus dem geschützten Phosphinsäureester 6 das Phosphinsäureester-hydrochlorid 11 in einer Ausbeute von 95%.

MS (FAB, NBA, m/e): 181 (M+H⁺).

### Beispiel 13

### Bis-(1-Amino-but-3-inyl)-phosphinsäure-ethylester-hydrochlorid (12)

In Analogie zum Beispiel 11 erhält man aus dem geschützten Phosphinsäureester 7 das Phosphinsäureester-hydrochlorid 12 in einer Ausbeute von 93%.

MS (FAB, NBA, m/e): 229 (M+H⁺).

### Beispiel 14

### Bis-(1-Amino-4-phenyl-but-3-enyl)-phosphinsäure-ethylester-hydrochlorid(13)

In Analogie zum Beispiel 11 erhält man aus dem geschützten Phosphinsäureester 8 das Phosphinsäureester-hydrochlorid 13 in einer Ausbeute von 90%.

MS (FAB, NBA, m/e): 385 (M+H⁺).

### Beispiel 15

### Bis-(1-Amino-3-brom-but-3-enyl)-phosphinsäure-ethylester-hydrochlorid (14)

In Analogie zum Beispiel 11 erhält man aus dem geschützten Phosphinsäureester 9 das Phosphinsäureester-hydrochlorid 14 in einer Ausbeute von 82%.

MS (FAB, NBA, m/e): 389, 391, 393 (M+H⁺).

### Beispiel 16

### Bis-(1-Amino-2-phenyl-ethyl)-phosphinsäure-hydrobromid (15)

Eine Lösung des bisalkylierten Phosphinsäuresters 5 (205 mg, 0,31 mmol) in 30-proz. Bromwasserstoff in Eisessig (11 ml) wurde für 25 Minuten auf eine Temperatur von 55-65°C erwärmt. Nach Einengen der Reaktionslösung unter vermindertem Druck und wiederholtem Koevaporieren mit Methanol und Toluol erhält man das Phosphinsäure-hydrobromid 15 (136 mg, 94% d.Th.).

¹H-NMR (δ/ppm/)(200 MHz, DMSO-d₆): 3,03 (4H, m, CH₂Ph; 3,95 (1H, m, CH); 4,21 (1H, m, CH); 7,20-7,45 (10H, m, H_{arom.}); 8,72 (6H, bs, NH₃+); MS (FAB, NBA, m/e): 305 (M+H+); 120 (100%).

### Beispiel 17

### Bis-(1-Amino-2-phenyl-ethyl)-phosphinsäure-hydrobromid (15)

In Analogie zum Beispiel 16 erhält man aus dem Phosphinsäurester-hydrochlorid 10 das Phosphinsäure-hydrobromid 15 in einer Ausbeute von 92%.

### Beispiel 18

### Bis-(N-tert-butoxycarbonyl-amonomethyl)-phosphinsäure-benzylester (16)

In Analogie zu Beispiel 3 wurde aus der Phosphinsäure 1 (1,910 g; 5,90 mmol) Benzylakhohol (1,25 ml; 12,15 mmol), Chlorameisensäure-iso-butyl-ester (0,90 ml; 6,89 mmol), Triethylamin (1,00 ml; 7,19 mmol) und N,N-Diemthylaminopyridin 870 mg; 0,57) mmol der Phosphinsäure-benzylester 16 (1,346 g, 55 % d. Th.) erhalten

¹H-NMR (δ/ppm)(200 MHu, CDCl₃): 1,44 (18 H,s, (CH₃)₃C); 3,28 (2H, dt, J 15 und 5 Hz, CH₂); 3,88 (2 H, m, CH₂); 5,13 (2 H, d, J 6 Hz, CH₂Ph); 5,25 (2 H, m, NH); 7,15-7,45 (5 H, m, H_{arom.});
MS (FAB, NBA, m/e): 415 (M+H⁺) (100 %); 359 (M-55).

### Beispiel 19

### Bis-(N-tert-butoxycarbonyl-aminomethyl)-phosphinsäure-methylester (17)

In Analogie zu Beispiel 3 wurde aus der Phosphinsäure 1 (8,365 g; 28,82 mmol) Methanol (5,50 ml; 135,78 mmol), Chlorameisensäure-iso-butyl-ester (3,75 ml; 28,69 mmol), Triethylamin (4,00 ml; 28,75 mmol) und N,N-Dimethylaminopyridin (330 mg; 2,70 mmol) der Phosphinsäureester 17 (2,570 g, 29 % d. Th.) erhalten.

¹H-NMR (δ/ppm)(200 MHz, CDCl₃): 1,44 (18 H, s, (CH₃)₃C); 3,25 (2 H, dt, J 15 und 5 Hz, CH₂); 3,79 (3 H, d, J 10 Hz, OCH₃); 3,84 (2 H, m, CH₂); 5,46 (2 H, m, NH);
MS (FAB, NBA, m/e): 339 (M+H⁺); 283 (M-55); 227 (100 %).

### Beispiel 20

### Bis-(aminomethyl-phosphinsäure-methylester-hydrochlorid (18)

Die tert-Butoxycarbonyl-Schutzgruppe des Phosphinsäure-methylesters 17 (2,470 g; 7,31 mmol) wird mit 3 N methanolischer Salzsäure (50 ml) in Analogie zu Beispiel 4 abgespalten. Man erhält das Phosphinsäure-methylester-hydrochlorid 18 (1,522 g; 99 % d. Th.).

¹H-NMR (δ/ppm)(200 MHz, D₂O): 3,72 (2 H, d, J 10 Hz, CH₂); 3,73 (2 H, d, J 10 Hz, CH₂); 3,96 (3 H, d, J 10 Hz, OCH₃);
MS (FAB, NBA, m/e): 139 (M+H⁺) (100 %); 110.

### Beispiel 21

### Bis-(Campher-imin-methyl)-phospinsäure-ethylester (21)

Eine Suspension von Bis-(aminomethyl)-phosphinsäure-ethylester-hydrochlorid (0,667 g; 2,96 mmol) und Campher-imin 80,895 g; 5,93 mmol, s. Beispiel 21a) in Methylenchlorid (100 ml) wird bei Raumtemperatur intensiv gerührt. Nach 65 Stunden wird das Reaktionsgemisch mit Wasser versetzt. Die wäßrige Phase wird mit Methylenchlorid extrahiert. Die vereinigte organische Phase wird getrocknet und unter vermindertem Druck eingeengt. Nach säulenchronmatographischer Reinigung an Kieselgel (Essigester/Methanol 95/5) erhält man die Campherverbindung 21 (0,969 g; 80 % d. Th.).

¹H-NMR (δ/ppm)(200 MHz, CDCl₃): 0,77 (6 H, s, CH₃); 0,91 (6 H, s, CH₃); 0,97 (6 H, s, CH₃); 1,16-2,06 (12 H, m, CH₂); 1,31 (3 H, t, J 6 Hz, CH₂CH₃); 2,47 (2 H, m, CH); 3,83 (4 H, m, PCH₂); 4,17 (2 H, dq, J 6 und 6 Hz, CH₂CH₃); ³¹P (δ/ppm)(109 MHz, CDCl₃): 47,27;
MS (FAB, m/e): 421 (M+H⁺) (100 %), 164 (25 %).

### Beispiel 21a

### (1R)-Capher-imin (20)

Zu einem intensiv gerührten Lösungsgemisch aus Campher-oxim (19, s. Beispiel 21b) (3,65 g; 21,86 mmol) in Ether (50 ml) und aus Natriumnitrit (3,65 g; 52,90 mmol) in Wasser (20 ml) wird langsam Schwefelsäure 81,1 ml; verdünnt mit 20 ml Wasser) zugegeben. Die Reaktionslösung wird bordeaux-braun. Nach 15 Minuten wird die organische Phase abgetrennt, getrocknet und unter vermindertem Druck eingeengt. Das so erhaltene feste Oxim-nitrimin wechselt unter einer Stickstoffatmosphäre die Farbe von gelb bis türkis, dabei zerfließend. In eine Suspension des Nitrimins in Tetrahydrofuran (THF) wird bei -15°C Ammoniak eingeleitet. Die hell, bernsteinklare Lösung wird nach 30 Minuten unter vermindertem Druck eingeengt. Der Rückstand wird in Methylenchlorid aufgenommen. Nach Filtration wird das Filtrat unter vermindertem Druck eingeengt. Man erhält das Campher-imin (20) als Rohprodukt quantitativ.

¹H-NMR (δ/ppm)(200 MHz, CDCl₃): 0,80 (3 H, s, CH₃); 0,93 (3 H, s, CH₃); 0,95 (3 H, s, CH₃); 1,23-2,57 (7 H, m, CH₂ + CH); 5,81 (1 H, br, s, NH). MS (Cl, m/e: 152 (M+H⁺) (100 %), 95 (15 %).

### Beispiel 21b

### (1R)-Campher-oxim (19)

Zu einer unter Rückfluß siedenden Lösung von (1R)(+)Campher (10,01 g; 65,86 mmol) und Hydroxylaminhydrochlorid 810,04 g; 144,46 mmol) in 150 ml Ethanol und 20 ml Wasser wird vorsichtig Natriumhydroxid (15,03 g; 375,50 mmol) hinzugegeben. Nach 7 Stunden wird die Reaktionslösung abgekühlt, mit Wasser verdünnt und filtriert. Das Filtrat wird mit Essigsäure auf einen pH von 5-6 gebracht. Nach erneuter Filtration erhält man das Oxim 19 83,81 g, 35 % d. Th.) (Fₚ 188-20°C).

¹H-NMR (δ/ppm)(200 MHz, CDCl₃): 0,81 (3 H,s, CH₃); 0,92 (3 H, s, CH₃); 1,00 (3 H, s, CH₃); 1,15-2,12 (6 H, m, CH₂); 2,55 (1 H, dt, J 17 und 3 Hz, CH); 7,82 (1 H, s, OH).

### Beispiel 22

### Bis-(1-[Campher-imin]-2-phenyl-ethyl)-phosphinsäure-ethylester (22) (Campher-imin-methyl)(1-[Campher-imin]-2-phenyl-ethyl)-phosphinsäure-ethylester (23)

Zu einer unter einer Argonatmosphäre gerührten Lösung der Campherverbindung 21 (0,320 g; 0,76 mmol) in abs. THF (30 ml) tropft man bei -78°C einer 1,50 N Butyllithiumlösung (1,40 ml; 2,10 mmol). Nach 5 Minuten wird Benzylbromid 80,37 ml; 3,12 mmol) dazugegeben. Nach 65 Stunden wird die Reaktionslösung mit einer gesättigten wäßrigen Ammoniumchlorid-Lösung versetzt und mit Essigester (EE) verdünnt. Die wäßrige Phase wird mit EE extrahiert. Dier vereinigte organische Phase wird getrocknet und unter vermindertem Druck eingeengt. Nach chromatographischer Reinigung (EE/Heptan 9/1) erhält man zwei bisalkylierte Verbindungen 22 (43 mg, 9 % d. Th.).und die monoalkylierte Verbindung 23 (131 mg, 34 % d. Th.).

### 22:

¹H-NMR (δ/ppm)(200 MHz, CDCl₃): 0,66-1,02 (18 H, m, CH₃); 1,20-2,46 (17 H, m, CH₂ + CH + CH₂CH₂CH₃); 2,88-3,58 (4 H, m CH₂Ph); 3,82-4,35 (4 H, m, PCH + CH₂CH₃); 7,10-7,28 (10 H, m, H_{arom.});
³¹P (δ/ppm)(109 MHz, CDCl₃); 48,55; 48,84;
MS (FAB, m/e): 601 (M+H⁺) (100 %), 567 (15 %), 254 (82 %).

### 23:

¹H-NMR (δ/ppm)(200 MHz, CDCl₃): 0,68 (3 H, s, CH₃); 0,78 (3 H, s, CH₃); 0,82 (3 H, s, CH₃); 0,92 (3 H, s, CH₃); 0,94 (3 H, s, CH₃); 0,97 (3 H, s, CH₃); 1,30-2,08 (15 H, m, CH₂ + CH₂CH₃); 2,27 (1 H, dd, J 16 Hz, CH); 2,46 (1 H, dd, J 16 Hz, CH); 3,08 (1 H, m α-CH₂Oh); 3,34 (1 H, m, β-CH₂Ph); 3,63-4,35 (5 H, m, PCH₂ + PCH + CH₂CH₃); 7,13-7,30 (5 H, m, H_{arom.});
³¹P (δ/ppm)(109 MHz, CDCl₃); 48,44; 48,95;
MS (FAB, m/e): 601 (M+H⁺) (100 %), 360 (5 %), 254 (38 %).

## Patentansprüche

1. Verfahren zur Herstellung von α- bzw. α,α'-substituierten Derivaten der Bis(aminomethyl)phosphinsäure der Formel I und deren saurer oder basischen Salze, 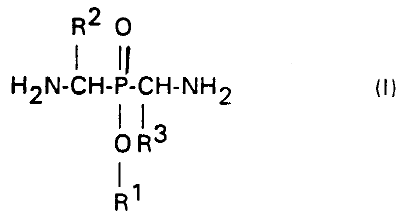 worin R¹ für R⁶ = C₁-C₁₈ Alkyl, C₂-C₁₈ Alkenyl, C₂-C₁₈ Alkinyl, C₆-C₁₂ Aryl, C₇-C₂₀ Arylalkyl steht und Aryl oder Arylalkyl ein oder mehrfach durch Fluor, Chlor, Brom, NO₂, CN, OH, COOH, C₁-C₆ Alkyl, C₁-C₆ Alkoxy, C(O)-O-(C₁-C₆)-Alkyl , C(O)-(C₁-C₆)-Alkyl , O-C(O)-(C₁-C₆)-Alkyl substituiert sein kann, und
R¹ darüber hinaus für Wasserstoff oder basische oder saure Salzreste der α-bzw. α,α'-substituierten Derivate der Bis(aminomethyl)phosphinsäure, steht R² und R³ gleich oder verschieden sind und unabhängig voneinander für Wasserstoff, C₁-C₁₈ Alkyl, C₂-C₁₈ Alkenyl, C₂-C₁₈ Alkinyl, C₆-C₁₂ Aryl oder C₇-C₂₂ Arylalkyl steht, wobei Alkyl, Alkenyl oder Alkinyl jeweils ein oder mehrfach durch Fluor, Chlor, Brom, NO₂, NH₂, CN, OH, COOH, C(O)-O-(C₁-C₆)-Alkyl , C(O)-(C₁-C₆)Alkyl , O-C(O)-(C₁-C₆)Alkyl oder C₁-C₆ Alkoxy substituiert sein können, und Aryl oder Arylalkyl ein oder mehrfach durch Fluor, Chlor, Brom, NO₂, CN, OH, COOH, C₁-C₆ Alkyl, C₁-C₆ Alkoxy, C(O)-O-(C₁-C₆)Alkyl, C(O)-(C₁-C₆) Alkyl , O-C(O)-(C₁-C₆) Alkyl substituiert sein kann, mit der Maßgabe, daß falls R² = Wasserstoff ist und R³ die obengenannte Bedeutung hat, R³ nicht ebenfalls für Wasserstoff stehen darf und umgekehrt,
dadurch gekennzeichnet, daß man
a) Bis(aminomethyl)phosphinsäure nach Einführung von Aminoschutzgruppen mit einer Verbindung der Formel R⁶-OH, worin R⁶ die unter R¹ genannte Bedeutung hat, verestert, oder
b) Bis(chlormethyl)phosphinsäurechlorid zunächst mit einer Verbindung der Formel R⁶-OH verestert, die so erhaltene Verbindung mit Benzylamin zum Bis(N-benzylaminomethyl)phosphinsäureester umsetzt,
dann nach Abspaltung der Aminoschutzgruppen der nach a) oder b) erhaltenen Verbindungen den gebildeten Bis(aminomethyl)phosphinsäureester der Formel IV 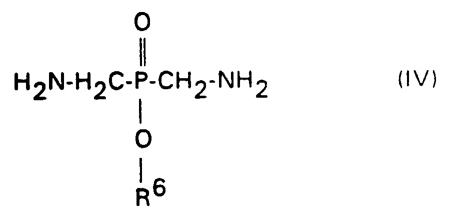 worin R⁶ wie oben definiert ist, mit einer Verbindung der Formel VI 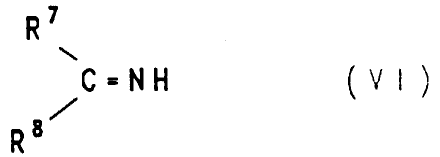 oder einer Verbindung der Formel VII 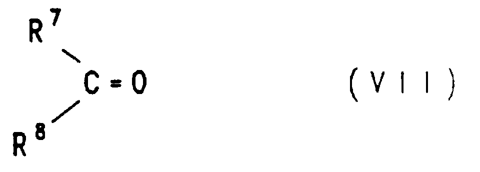 worin R⁷ und R⁸ gleich oder verschieden sind und für Wasserstoff, C₁-C₂₀-Alkyl, C₂-C₂₀-Alkenyl, C₂-C₂₀-Alkinyl oder C₆-C₁₂-Aryl stehen oder R⁷ und R⁸ gemeinsam ein Ringsystem bilden, unter wasserabscheidenden Bedingungen zu einer Verbindung der Formel V 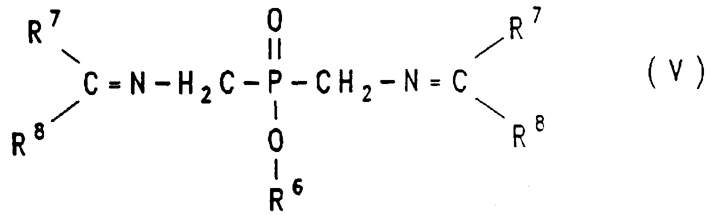 umsetzt, worin R⁶, R⁷ und R⁸ die obengenannte Bedeutung haben, man
e₁) die Verbindung der Formel V, nach Umsetzung mit 2-3 Equivalenten Base, mit 2-5 Equivalenten einer Verbindung der Formel R³-X, worin R³ die obengenannte Bedeutung, ausgenommen Wasserstoff, hat und X für eine Abgangsgruppe steht, zu einer Verbindung der Formel VIII 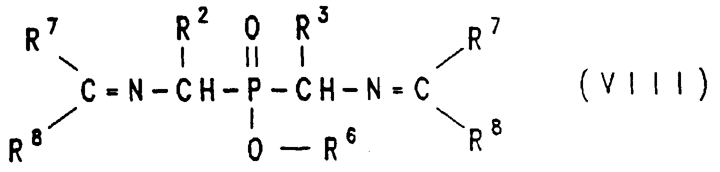 umsetzt, worin R² = R³ ist und R³, R⁶, R⁷ und R⁸ die obengenannte Bedeutung, ausgenommen R³ = Wasserstoff, haben, oder man
e₂) analog e₁) vorgeht, jedoch unter Einsatz von 1 bis 1, 2 Equivalenten Base und 1 bis 1, 2 Equivalenten R³-X, wobei man eine Verbindung der Formel VIII erhält, worin R² Wasserstoff bedeutet und R³, R⁶, R⁷ und R⁸ die obengenannte Bedeutung, ausgenommen R³ = Wasserstoff, haben, man gegebenenfalls
e₃) die in e₂) erhaltene Verbindung der Formel VIII, nach Umsetzung mit weiteren 1 bis 1, 2 Equivalenten einer Base, mit 1 bis 1, 2 Equivalenten einer Verbindung der Formel R²-X, worin R² und X die obengenannte Bedeutung, ausgenommen R² = Wasserstoff, haben, zu einer Verbindung der Formel VIII, worin R², R³, R⁶, R⁷ und R⁸ die obengenannte Bedeutung, ausgenommen R² und R³ = Wasserstoff haben, umsetzt, oder man
f₁) die Verbindung der Formel V mit einem Katalysator der Formel
R⁹R¹⁰R¹¹R¹²NZ, 2 bis 3 Equivalenten einer Verbindung der Formel R³-X und 2-25 Equivalenten Alkali- oder Erdalkalimetallhydroxid, wobei N für Stickstoff oder Phosphor, Z für ein Anion eines anorganischen Salzes R⁹, R¹⁰, R¹¹ und R¹² gleich oder verschieden sind und unabhängig voneinander für C₁-C₁₀-Alkyl, C₂-C₂₀-Alkenyl, C₂-C₂₀-Alkinyl, C₆-C₁₂-Aryl oder C₇-C₂₀-Arylalkyl stehen und R³ und X wie in e₁) definiert sind, in Gegenwart eines mit Wasser nicht mischbaren Lösemittels zu einer Verbindung der Formel VIII, worin R² = R³ ist und R³, R⁶, R⁷ und R⁸ die obengenannte Bedeutung, ausgenommen R³ = Wasserstoff, haben, umsetzt, oder man
f₂) analog f₁₎ vorgeht, jedoch unter Einsatz von 2 - 10 Equivalenten eines Alkali- oder Erdalkalimetallhydroxids und 1 bis 1, 2 Equivalenten R³-X, wobei man eine Verbindung der Formel VIII erhält, worin R² für Wasserstoff steht und R³, R⁶, R⁷ und R⁸ die obengenannte Bedeutung, ausgenommen R³ = Wasserstoff, haben, man gegebenenfalls
f₃) die nach f₂) erhaltene Verbindung der Formel VIII unter den in f₁) genannten Bedingungen mit R²-X anstelle von R³-X zu einer Verbindung der Formel VIII, in der R², R³, R⁶, R⁷ und R⁸ die obengenannte Bedeutung, ausgenommen R² und R³ = Wasserstoff, haben, umsetzt, wobei jedoch 2 bis 10 Equivalente des Alkali- oder Erdalkalimetallhydroxids und 1 bis 1,2 Equivalente R²-X eingesetzt werden, oder man
g) analog f₁) - f₃) vorgeht, jedoch ohne Verwendung eines Lösemittels und mit Methyltrioctylammoniumchlorid anstelle von R⁹ R¹⁰R¹¹R¹²NZ, wobei man eine Verbindung der Formel VIII mit den in f₁) - f₃) genannten Bedeutungen erhält,
h) die nach e), f) oder g) erhaltene Verbindung der Formel VIII mit 5-15 % wäßriger HCI oder HBr zu einer Verbindung der Formel I, worin R¹ für R⁶ steht und R² und R³ die in e), f) oder g) genannte Bedeutung haben, umsetzt, oder man
i) die nach e), f) oder g) erhaltene Verbindung der Formel VIII oder gegebenenfalls die nach h) erhaltene Verbindung der Formel I mit 30 % HBr oder HCI in Eisessig zu einer Verbindung der Formel I, worin R² und R³ die in e), f) oder g) genannte Bedeutung haben und R¹ wie oben definiert, jedoch R¹ ungleich R⁶, ist, umsetzt.

## Claims

1. A process for the preparation of α- or α,α'- substituted derivatives of bis(aminomethyl)phosphinic acid of the formula I and their acid or basic salts 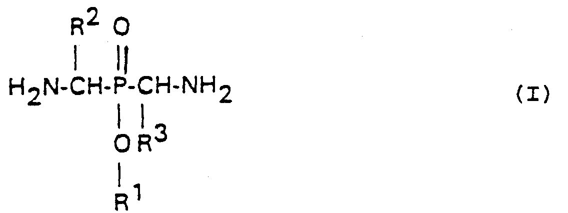 in which R¹ is R⁶ = C₁-C₁₈ alkyl, C₂-C₁₈ alkenyl, C₂-C₁₈ alkynyl, C₆-C₁₂ aryl or C₇-C₂₀ arylalkyl and aryl or arylalkyl may be mono- or polysubstituted by fluorine, chlorine, bromine, NO₂, CN, OH, COOH, C₁-C₆ alkyl, C₁-C₆ alkoxy, C(O)-O-(C₁-C₆) alkyl, C(O)-(C₁-C₆) alkyl or O-C(O)-(C₁-C₆) alkyl, and
R¹ is also hydrogen or basic or acid salt radicals of the α- or α,α'-substituted derivatives of bis (aminomethyl) - phosphinic acid,
R² and R³ are identical or different and are, independently of each other, hydrogen, C₁-C₁₈ alkyl, C₂-C₁₈ alkenyl, C₂-C₁₈ alkynyl, C₆-C₁₂ aryl or C₇-C₂₂ arylalkyl, where alkyl, alkenyl or alkynyl may each be mono- or polysubstituted by fluorine, chlorine, bromine, NO₂, NH₂, CN, OH, COOH, C(O)-O-(C₁-C₆) alkyl, C(O)-(C₁-C₆) alkyl, O-C(O)-(C₁-C₆) alkyl or C₁-C₆ alkoxy, and aryl or arylalkyl may be mono- or polysubstituted by fluorine, chlorine, bromine, NO₂, CN, OH, COOH, C₁-C₆ alkyl, C₁-C₆ alkoxy, C(O)-O-(C₁-C₆) alkyl, C(O)-(C₁-C₆) alkyl or O-C(O)-(C₁-C₆) alkyl, with the proviso that if R² = hydrogen and R³ has the abovementioned meaning, R³ may not also be hydrogen, and vice versa,
which comprises
a) esterifying bis(aminomethyl)phosphinic acid, after introducing amino protective groups, with a compound of the formula R⁶-OH, in which R⁶ has the meaning mentioned under R¹, or
b) initially esterifying bis(chloromethyl)phosphinyl chloride with a compound of the formula R⁶-OH, and reacting the compound thus obtained with benzylamine to give the bis(N-benzylaminomethyl) phosphinic ester,
then, after removing the amino protective groups from the compounds obtained by a) or b), reacting the bis(aminomethyl)phosphinic ester of the formula IV 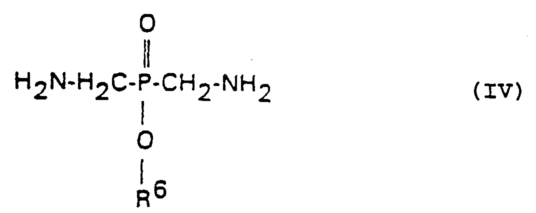 which has been formed, and in which R⁶ is as defined above, with a compound of the formula VI 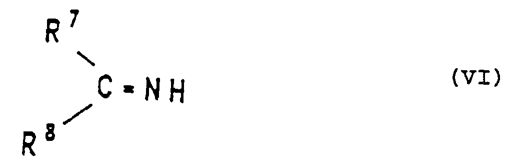 or a compound of the formula VII 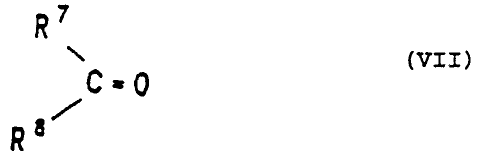 in which R⁷ and R⁸ are identical or different and are hydrogen, C₁-C₂₀ alkyl, C₂-C₂₀ alkenyl, C₂-C₂₀ alkynyl or C₆-C₁₂ aryl or R⁷ and R⁸ together form a ring system, under conditions in which water is separated off, to give a compound of the formula V 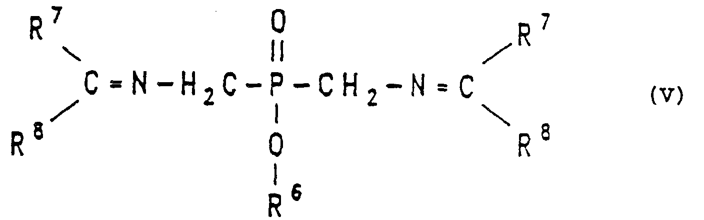 in which R⁶, R⁷ and R⁸ have the abovementioned meaning,
e₁) reacting the compound of the formula V, after reaction with 2-3 equivalents of base, with 2-5 equivalents of a compound of the formula R³-X, in which R³ has the abovementioned meaning, except for hydrogen, and X is a leaving group, to give a compound of the formula VIII 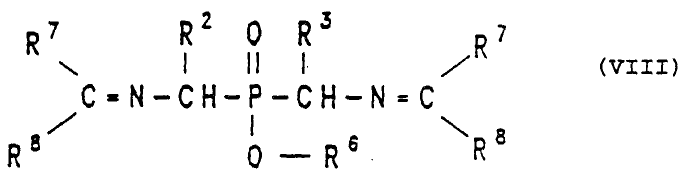 in which R² = R³ and R³, R⁶, R⁷ and R⁸ have the abovementioned meaning, except for R³ = hydrogen, or
e₂) proceeding in an analogous manner to e₁), but using 1 to 1.2 equivalents of base and 1 to 1.2 equivalents of R³-X, thereby obtaining a compound of the formula VIII in which R² is hydrogen and R³, R⁶, R⁷ and R⁸ have the abovementioned meaning, except for R³ = hydrogen, optionally
e₃) reacting the compound of the formula VIII obtained in e₂), after reaction with a further 1 to 1.2 equivalents of a base, with 1 to 1.2 equivalents of a compound of the formula R²-X, in which R² and X have the abovementioned meaning, except for R² = hydrogen, to give a compound of the formula VIII in which R², R³, R⁶, R⁷ and R⁸ have the abovementioned meaning, except for R² and R³ = hydrogen, or
f₁) reacting the compound of the formula V with a catalyst of the formula R⁹R¹⁰R¹¹R¹²NZ, 2 to 3 equivalents of a compound of the formula R³-X and 2 - 25 equivalents of an alkali metal hydroxide or alkaline earth metal hydroxide, where N is nitrogen or phosphorus, Z is an anion of an inorganic salt, R⁹, R¹⁰, R¹¹ and R¹² are identical or different and, independently of each other, are C₁-C₁₀ alkyl, C₂-C₂₀ alkenyl, C₂-C₂₀ alkynyl, C₆-C₁₂ aryl or C₇-C₂₀ arylalkyl, and R³ and X are as defined in e₁), in the presence of a water-immiscible solvent, to give a compound of the formula VIII in which R² = R³ and R³, R⁶, R⁷ and R⁸ have the abovementioned meaning, except for R³ = hydrogen, or
f₂) proceeding in an analogous manner to f₁), but using 2 - 10 equivalents of an alkali metal or alkaline earth metal hydroxide and 1 to 1.2 equivalents of R³-X, thereby obtaining a compound of the formula VIII in which R² is hydrogen and R³, R⁶, R⁷ and R⁸ have the abovementioned meaning, except for R³ = hydrogen, optionally
f₃) reacting the compound of the formula VIII obtained by f₂), under the conditions mentioned in f₁), with R²-X instead of R³-X, to give a compound of the formula VIII in which R², R³, R⁶, R⁷ and R⁸ have the abovementioned meaning, except for R² and R³ = hydrogen, using, however, 2 to 10 equivalents of the alkali metal or alkaline earth metal hydroxide and 1 to 1.2 equivalents of R²-X, or
g) proceeding in an analogous manner to f₁) - f₃), but using no solvent, and with methyltrioctylammonium chloride instead of R⁹R¹⁰R¹¹R¹²NZ, thereby obtaining a compound of the formula VIII with the meanings mentioned in f₁) - f₃),
h) reacting the compound of the formula VIII obtained by e), f) or g) with 5-15% strength aqueous HCl or HBr to give a compound of the formula I, in which R¹ is R⁶ and R² and R³ have the meaning mentioned in e), f) or g), or
i) reacting the compound of the formula VIII obtained by e), f) or g) or optionally the compound of the formula I obtained by h) with 30% strength HBr or HCl in glacial acetic acid to give a compound of the formula I in which R² and R³ have the meaning mentioned in e), f) or g) and R¹ is defined as above, R¹, however, not being R⁶.

## Revendications

1. Procédé de fabrication de dérivés de l'acide bis(aminométhyl)-phosphinique de formule I substitués en α ou en α,α' et de leurs sels acides ou basiques, 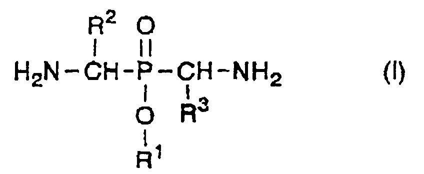 dans lesquels R¹ représente R⁶ = un groupe alkyle en C₁-C₁₈, alcényle en C₂-C₁₈, alcynyle en C₂-C₁₈, aryle en C₈-C₁₂, arylalkyle en C₇-C₂₀, le groupe aryle ou arylalkyle pouvant être substitué une ou plusieurs fois par un atome de fluor, de chlore, de brome, par les groupes NO₂, CN, OH, COOH, alkyle en C₁-C₆, alcoxy en C₁-C₆, C(O)-O-alkyle (C₁-C₆), C(O)-alkyle (C₁-C₆), O-C(O)-alkyle (C₁-C₆), et
R¹ représente de plus un atome d'hydrogène ou un reste salin basique ou acide d'un dérivé substitué en α ou en α,α' l'acide bis(aminométhyl)-phosphinique,
R² et R³ sont identiques ou différents, et représentent, indépendamment l'un de l'autre, un atome d'hydrogène, un groupe alkyle en C₁-C₁₈, alcényle en C₂-C₁₈, alcynyle en C₂-C_{18,} aryle en C₆-C₁₂ ou arylalkyle en C₇-C₂₂, le groupe alkyle, alcényle ou alcynyle pouvant être substitué une ou plusieurs fois par un atome de fluor, de chlore, de brome, par NO₂, NH₂, CN, OH, COOH, un groupe C(O)-O-alkyle (C₁-C₆), C(O)-alkyle (C₁-C₆), O-C(O)-alkyle (C₁-C₆) ou alcoxy en C₁-C₆, le groupe aryle ou arylalkyle pouvant être substitué une ou plusieurs fois par un atome de fluor, de chlore, de brome, par NO₂, CN, OH, COOH, un groupe alkyle en C₁-C₆, alcoxy en C₁-C₆, C(O)-O-alkyle (C₁-C₆), C(O)-alkyle (C₁-C₆), O-C(O)-alkyle (C₁-C₆), sous réserve que, dans le cas où R² = atome d'hydrogène et R³ représente un des groupes cités ci-dessus, R³ n'est pas simultanément un atome d'hydrogène et inversement,
caractérisé en ce que
a) on estérifie l'acide bis(aminométhyl)phosphinique après introduction de groupes aminoprotecteurs avec un composé de formule R⁶-OH dans lequel R⁶ a la signification donnée pour R¹ ou bien
b) on estérifie d'abord le chlorure de l'acide bis(chlorométhyl)phosphinique avec un composé de formule R⁶-OH et l'on fait réagir le composé ainsi obtenu avec de la benzylamine pour former un ester de l'acide bis(N-benzylaminométhyl)phosphinique,
puis après élimination des groupes aminoprotecteurs du composé obtenu selon a) ou b), on fait réagir l'ester de l'acide bis(aminométhyl)phosphinique obtenu, de formule IV 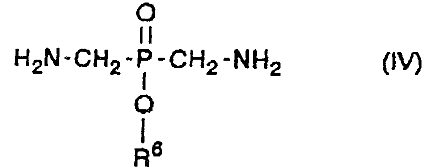 dans lequel R⁶ est défini comme ci-dessus, avec un composé de formule VI 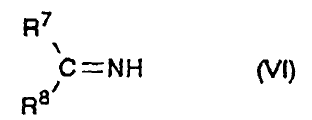 ou un composé de formule VII 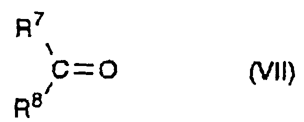 dans lesquels R⁷ et R⁸ sont identiques ou différents et représentent un atome d'hydrogène, un groupe alkyle en C₁-C₂₀, alcényle en C₂-C₂₀, alcynyle en C₂-C₂₀ ou aryle en C₆-C₁₂ ou bien R⁷ et R⁸ représentent ensemble un système cyclique, pour obtenir, en éliminant l'eau formée, un composé de formule V 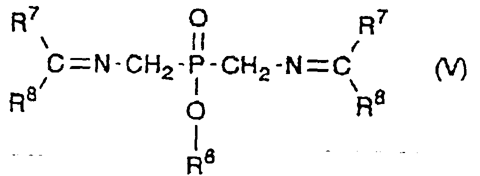 dans lequel R⁶, R⁷ et R⁸ ont les significations données ci-dessus.
e₁) on fait réagir le composé de formule V, après réaction avec 2 à 3 équivalents de base, avec 2 à 5 équivalents d'un composé de formule R³-X dans lequel R³ a la signification donnée ci-dessus, à l'exception de l'atome d'hydrogène et X représente un groupe partant, pour former un composé de formule VIII 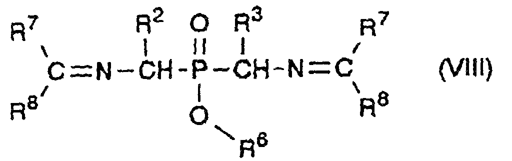 dans lequel R² = R³ et R³, R⁶, R⁷ et R⁸ ont les significations données ci-dessus, à l'exception de R³ = atome d'hydrogène, ou bien
e₂) on procède de façon analogue à e₁), mais en utilisant 1 à 1,2 équivalents de base et 1 à 1,2 équivalents de R³-X et l'on obtient un composé de formule VIII dans lequel R² est un atome d'hydrogène et R³, R⁶, R⁷ et R⁸ ont les significations données ci-dessus, à l'exception de R³ = atome d'hydrogène,
e₃) le cas échéant, on fait réagir le composé de formule VIII obtenu selon e₂), après réaction avec 1 à 1,2 équivalents supplémentaires de base avec 1 à 1,2 équivalents d'un composé de formule R²-X dans lequel R² et X ont les significations données ci-dessus, à l'exception de R² = atome d'hydrogène, pour former un composé de formule VIII dans lequel R², R³, R⁶, R⁷ et R⁸ ont les significations données ci-dessus, à l'exception de R² et R³ = atome d'hydrogène, ou bien
f₁) on fait réagir le composé de formule V avec un catalyseur de formule R⁹R¹⁰R¹¹R¹²NZ, 2 à 3 équivalents d'un composé de formule R³-X et 2 à 25 équivalents d'hydroxyde d'un métal alcalin ou alcalinoterreux, où N représente un atome d'azote ou de phosphore, Z un anion d'un sel anorganique, R⁹, R¹⁰, R¹¹ et R¹² sont identiques ou différents, et représentent, indépendamment les uns des autres, un groupe alkyle en C₁-C₁₀, alcényle en C₂-C₂₀, alcynyle en C₂-C₂₀, aryle en C₆-C₁₂ ou arylalkyle en C₇₋C₂₀ et R³ et X sont définis comme en e₁), en présence d'un solvant non miscible à l'eau, pour obtenir un composé de formule VIII dans lequel R² = R³ et R³, R⁶, R⁷ et R⁸ ont les significations données ci-dessus, à l'exception de R³ = atome d'hydrogène, ou bien
f₂) on procède de façon analogue à f₁), mais en utilisant 2 à 10 équivalents d'un hydroxyde de métal alcalin ou alcalinoterreux et 1 à 1,2 équivalents de R³-X, pour obtenir un composé de formule VIII dans lequel R² = atome d'hydrogène et R³, R⁶, R⁷ et R⁸ ont les significations données ci-dessus, à l'exception de R³ = atome d'hydrogène, ou bien, le cas échéant,
f₃) on fait réagir le composé de formule VIII obtenu selon f₂), dans les condition décrites pour f₁) avec R²-X à la place de R³-X pour obtenir un composé de formule VIII dans lequel R², R³, R⁶, R⁷ et R⁸ ont la signification donnée précédemment, à l'exception de R² et R³ = atome d'hydrogène, en utilisant 2 à 10 équivalents d'un hydroxyde de métal alcalin ou alcalinoterreux et 1 à 1,2 équivalents de R²-X, ou bien,
g) on procède de façon analogue à f₁) - f₃), mais sans utiliser de solvant et avec du chlorure de méthyltrioctylammonium à la place de R⁹R¹⁰R¹¹R¹²NZ, et on obtient un composé de formule VIII avec les significations données en f₁) à f₃),
h) on fait réagir les composés de formule VIII obtenus selon e), f) ou g) avec du HCl ou du HBr aqueux à 5 - 15 % pour obtenir un composé de formule I dans lequel R¹ représente R⁶ et R² et R³ ont les significations données en e), f) ou g), ou bien
i) on fait réagir les composés de formule VIII obtenus selon e), f) ou g) ou, le cas échéant, les composés de formule I obtenus selon h), avec du HBr ou du HCl à 30 % dans l'acide acétique glacial, pour obtenir un composé de formule I dans lequel R² et R³ ont les significations données en e), f) ou g) et R¹ est défini ci-dessus, mais R¹ est différent de R⁶.
